Europäisches Patentamt

European Patent Office

Office européen des brevets

(19)

(11) Publication number: **0 022 885**
**B1**

(12) **EUROPEAN PATENT SPECIFICATION**

(45) Date of publication of patent specification: **28.03.84**

(51) Int. Cl.³: **C 07 D 213/61, C 07 B 9/00**

(21) Application number: **79301442.4**

(22) Date of filing: **20.07.79**

(54) Process for recovering 2,3,5,6-tetrachloropyridine from mixtures of chlorinated pyridines.

(43) Date of publication of application:
28.01.81 Bulletin 81/4

(45) Publication of the grant of the patent:
28.03.84 Bulletin 84/13

(84) Designated Contracting States:
BE DE FR GB IT NL

(56) References cited:
GB - A - 991 526
US - A - 3 538 100
US - A - 3 668 209
US - A - 3 993 654

CHEMICAL ABSTRACTS, Vol. 65, 1966
Columbus, Ohio, USA

(73) Proprietor: THE DOW CHEMICAL COMPANY
Dow Center 2030 Abbott Road Post Office Box
1967
Midland Michigan 48640 (US)

(72) Inventor: Perettie, Donald Joseph
117 Poinciana Street
Lake Jackson, Brazoria Texas (US)
Inventor: Dean, Norman Leroy
103 Mulberry
Lake Jackson, Brazoria Texas (US)

(74) Representative: Baverstock, Michael George
Douglas et al,
BOULT, WADE & TENNANT 27 Furnival Street
London, EC4A 1PQ (GB)

Courier Press, Leamington Spa, England.

**0 022 885**

Process for recovering 2,3,5,6-tetrachloropyridine from mixtures of chlorinated pyridines

This invention is directed to a process for recovering substantially pure 2,3,5,6-tetrachloropyridine from mixtures of chlorinated pyridines.

Highly chlorinated pyridine compounds, such as 2,3,5,6-tetrachloropyridine, are known to be useful as pesticides or as intermediates for the preparation of other compounds having pesticidal properties. The various processes used to prepare chlorinated pyridines and especially 2,3,5,6-tetrachloropyridine have generally resulted in producing product mixes containing many chlorinated compounds including 2,3,4,5- and 2,3,4,6-tetrachloropyridine, the two position isomers of the desired 2,3,5,6-tetrachloropyridine. It has been particularly difficult to separate 2,3,5,6-tetrachloropyridine from its two position isomers by conventional economical techniques such as distillation since the boiling points of these three materials are so close. It has therefore been necessary to resort to more costly, difficult and time-consuming procedures of selective crystallization and multistage and/or multiplate distillation.

US—A—3,668,209 discloses a method for separating halogen substituted pyridines having an unhindered hydrogen atom in a position alpha or beta to the ring nitrogen from closely related halogen substituted pyridines which lack such a hydrogen atom. The separation is carried out by contacting a mixture of the pyridines with sulfuric acid or an alkaline sulfonic acid in the presence of a solvent for those species not possessing an unhindered hydrogen atom followed by a hydrolysis step.

It has now been found that both 2,3,4,5- and 2,3,4,6-tetrachloropyridine are converted to pentachloropyridine at a much more rapid rate than 2,3,5,6-tetrachloropyridine when they are chlorinated in the presence of certain acid catalysts. Because of this difference in reaction rates, both 2,3,4,5- and 2,3,4,6-tetrachloropyridine can be substantially converted to pentachloropyridine prior to substantial conversion of any 2,3,5,6-tetrachloropyridine which might be in admixture with the above isomers. The separation of 2,3,5,6-tetrachloropyridine from pentachloropyridine is a rather easy procedure.

Accordingly, the present invention provides a process for recovering substantially pure 2,3,5,6-tetrachloropyridine from a mixture of chlorinated pyridines consisting of 2,3,5,6-tetrachloropyridine, at least one of 2,3,4,5-tetrachloropyridine and 2,3,4,6-tetrachloropyridine and, optionally, pentachloropyridine, and no more than minor amounts, if any, of monochloropyridine, 3,5-dichloropyridine, 2,3,5-trichloropyridine and 2,3,6-trichloropyridine and no other chloropyridine compounds, which process comprises (a) reacting the mixture of chlorinated pyridines in liquid phase optionally in an inert solvent at a temperature above the melting point of the mixture under atmospheric or superatmospheric pressure with chlorine in the presence of up to 10 weight percent based on the weight of the chloropyridine reactant mixture of a catalyst which is o-phosphoric acid, boron trifluoride, aluminum chloride, aluminum oxychloride, chromium trichloride, ferric chloride, ruthenium chloride, aluminum bromide, aluminum oxybromide, chromium tribromide, ferric bromide, ruthenium bromide, aluminum oxide, ferric oxide, ferric acetylacetonate, antimony pentachloride, optionally supported on a carrier, to obtain a mixture consisting essentially of 2,3,5,6-tetrachloropyridine and pentachloropyridine and (b) recovering the 2,3,5,6-tetrachloropyridine by conventional techniques.

There are several commercially significant advantages of the present process over prior art procedures. First, the process of the invention results in a mixture consisting essentially of symmetrical tetrachloropyridine and pentachloropyridine which is easily separated by conventional techniques (e.g., distillation). Second, pentachloropyridine is known to be a starting material for use in reductive reactions leading to tetrachloropyridines and the pentachloropyridine produced in the present process can be recovered and recycled to generate additional symmetrical tetrachloropyridine. Third, the present process is a straightforward facile process which obviates the need of expensive, sophisticated distillation apparatus or selective crystallization apparatus to recover symmetrical tetrachloropyridine from a mixture of close boiling position isomers of tetrachloropyridine or a mixture of polychlorinated pyridines. Significant capital savings as well as reduced operating expenditures are thus substantially achieved. These and other advantages will be readily apparent to those skilled in the art of manufacturing symmetrical tetrachloropyridine.

Normally, commercial routes of producing symmetrical tetrachloropyridine result in a mixture of tri-, tetra- and pentachloropyridines. All of these mixtures normally melt at elevated temperatures (e.g., 90°C—125°C) and the molten mixture is used as the reaction medium in the present process. The catalyst, which is normally a solid can be employed as such (unsupported) or the catalyst can be supported on a carrier. Representative carriers include, for example, molecular sieves, gamma alumina, silica silica gel, silica-alumina, and graphite.

The preferred compounds are ferric chloride and aluminum chloride. The covalent metal halides can be used *per se* (which is the desired embodiment) or they can be generated *in situ* by adding the metal to the reaction mixture. For example, iron or aluminium can be added to the reaction mixture and ferric chloride and aluminium chloride will be generated *in situ*.

The catalysts are used in amounts of up to 10 weight percent, based on the weight of the chloropyridine reactant mixture. Preferably, however, the catalyst is included in amounts of from 0.05 to 0.5 weight percent. The catalysts are quite effective and generally the lesser amount is satisfactory

2

and facilitates removal of the catalyst at the end of the reaction.

The reaction may be conducted by contacting the chloropyridine mixtures with chlorine in the presence of the catalyst by a slurry technique or by contacting the mixture with the catalyst over a static bed or a fluidized bed of the catalyst. The process may be conducted in a batchwise manner, a continuous manner of a cyclic batch manner in which the pentachloropyridine and symmetrical tetrachloropyridine are removed leaving the catalyst in a distillation residue or in a quantity of the reaction mixture in the reaction vessel and fresh reactants then merely introduced into the reaction vessel. In this manner, the catalyst is recovered and re-used. The reaction is conducted at elevated temperatures normally above the melting point of the chloropyridine mixture. Desirable rates of reaction have been achieved at temperatures of from 150°C to 300°C although somewhat higher or lower temperatures could be used, if desired. Preferred rates of reaction have been observed at temperatures of from 200°C to 250°C.

The reaction can be conducted at atmospheric or superatmospheric pressure (e.g., 15—75 psig (2—6 atm) or higher) but superatmospheric pressures are normally desired. As is well known, chlorine is a gas and the solubility of chlorine in the chloropyridine mixtures is enhanced by use of superatmospheric pressures. This superatmospheric pressure may be provided with chlorine or an inert gas (e.g., nitrogen or HCl) but is normally provided by chlorine gas.

The process may be conducted in the presence of an inert solvent, if desired, but is preferably conducted neat. Suitable inert solvents include chlorinated hydrocarbons such as for example methyl chloroform, and carbon tetrachloride.

The following examples will further illustrate the invention.

Experimental

The following experiments were conducted in a glass reaction vessel at atmospheric pressure and in a high pressure stainless steel autoclave at elevated pressures. The analysis of the reaction mixture and end products was accomplished by vapor phase chromatography.

Examples 1—5

A mixture of chlorinated pyridines was charged to an externally heated glass reactor vessel equipped with a mechanical stirrer, reflux condenser and a glass sparger and the mixture heated until molten. The mixture of chloropyridines included some or all of the following compounds in various amounts: 3,5-Dichloropyridine, 2,3,5-trichloropyridine, 2,3,6-trichloropyridine, pentachloropyridine and the three-position isomers of tetrachloropyridine. Ferric chloride was added to this molten mixture with stirring. The molten mixture was then heated to approximately 200°C and chlorine was sparged into the reaction mixture. The course of the reaction was followed by taking analytical samples of the reaction mixture from time to time and followed the disappearance of the undesirable isomers in the gas chromatograph. The data from these examples (runs) are summarized in Table I below.

## TABLE I

### Influence of Catalyst Concentration at Atmospheric Pressure

| Ex. | Wt. % Catalyst | Feed, % Chloropyridine Isomers | | | | | Time Hrs. | % Conversion | | | | | |
|---|---|---|---|---|---|---|---|---|---|---|---|---|---|
| | | 3,5 | 2,3,5 | 2,3,6 | Tetras | Penta | | 3,5 | 2,3,5 | 2,3,6 | 2,3,4,5 | 2,3,4,6 | 2,3,5,6 |
| 1 | 0 | — | — | — | 73.37 | 25.36 | 4 | — | — | — | 6.04 | 7.87 | 5.22 |
| 2 | 0.05 | — | — | — | 73.43 | 25.21 | 6 | — | — | — | 17.47 | 56.59 | 4.37 |
| 3 | 0.50 | — | — | — | 73.71 | 24.61 | 3 | — | — | — | 12.66 | 100.0 | 7.06 |
| 4 | 3.21 | — | — | — | 73.22 | 25.66 | 2 | — | — | — | 65.81 | 100.0 | 13.49 |
| 5* | 2.94 | 0.096 | 2.834 | 0.662 | 67.23 | 23.19 | 5 | 100.0 | 77.98 | 100.0 | −143.0 | 97.4 | −0.1 |

### Tetrachloropyridine Isomer Distribution in Percent of Total Tetras

| Ex. | Initial | | | Final | | |
|---|---|---|---|---|---|---|
| | 2,3,4,5 | 2,3,4,6 | 2,3,5,6 | 2,3,4,5 | 2,3,4,6 | 2,3,5,6 |
| 1 | 0.126 | 2.673 | 97.20 | 0.125 | 2.600 | 97.27 |
| 2 | 0.121 | 2.664 | 97.21 | 0.106 | 1.228 | 98.67 |
| 3 | 0.115 | 2.621 | 97.26 | 0.111 | — | 98.87 |
| 4 | 0.123 | 2.699 | 97.18 | 0.050 | — | 99.95 |
| 5* | 0.140 | 2.86 | 97.0 | 0.350 | 0.076 | 99.57 |

*In this Run, 3,5-dichloropyridine was converted to 2,3,5-trichloropyridine which was converted to both 2,3,4,5- and 2,3,5,6-tetrachloropyridine and pentachloropyridine. 2,3,6-Trichloropyridine was converted to 2,3,5,6-tetrachloropyridine and pentachloropyridine. Additional reaction time would increase pentachloropyridine production and give substantially complete chlorination of 2,3,5-trichloropyridine, 2,3,4,5- and 2,3,4,6-tetrachloropyridine.

The negative numbers under "percent conversion" in Table I means that that particular isomer was being produced during the course of the reaction. The positive numbers, on the other hand, indicate that that particular isomer was being consumed during the course of the reaction and leading to reduced amounts of that material in the final product. The reader will note the very desirable ratio of symmetrical tetrachloropyridine to the other position isomers thereof in the final product. No di- or trichloropyridines were obsered in the final product (except run 5 as noted above). Final product thus consisted essentially of pentachloropyridine and symmetrical tetrachloropyridine. This mixture was resolved into pentachloropyridine and symmetrical tetrachloropyridine (along with any minor amounts of tetrachloropyridine positon isomers) by distillation under reduced pressure.

The dashes in Table I means that none of that particular isomer was present in either the feed or the final reaction product.

Examples 6—18

In this series of experiments, a mixture of tetra- and pentachloropyridines was charged to a high pressure stainless steel autoclave, the reaction mixture melted, and the amounts of $FeCl_3$-catalyst indicated in Table II added. The autoclave was subsequently sealed, heated to reaction temperature, and pressurized with a pad of chlorine entering through a top valve. Once pressurized, chlorine gas was then continuously sparged through the reaction mixture with stirring and analytical samples taken during the course of the reaction. The data from the these experiments are summarized in Table II.

## TABLE II

Experiments with $FeCl_3$ as Catalyst at Elevated Pressures
Influence of Catalyst Concentrate (Ex. 6–8), Pressure (Ex. 9–11) and Temperature (Ex. 12–18)

| | | Feed | | | Run Conditions | | | Tetrachloropyridine Isomers In Product | | | | | |
| | | Percent Tetrachloro-pyridine Isomers | | | | | | Distribution In Product, Percent | | | Percent Conversion | | |
| Ex. | Wt. % Catalyst* | 2,3,4,5 | 2,3,4,6 | 2,3,5,6 | Time hrs. | Temp °C | Pressure atm | 2,3,4,5 | 2,3,4,6 | 2,3,5,6 | 2,3,4,5 | 2,3,4,6 | 2,3,5,6 |
|---|---|---|---|---|---|---|---|---|---|---|---|---|---|
| 6 | 0.05 | 0.136 | 2.676 | 97.19 | 1.75 | 206 | 4.74 | 0.082 | 0.706 | 99.21 | 40.71 | 74.13 | — |
| 7 | 0.50 | 0.127 | 2.630 | 97.243 | 1.75 | 202 | 4.46 | 0.093 | — | 99.91 | 30.38 | 100.0 | 2.32 |
| 8 | 5.03 | 0.045 | 1.68 | 98.28 | 0.75 | 201 | 4.33 | 0.047 | — | 99.95 | 5.14 | 100.0 | 7.63 |
| 9 | 0.50 | 0.027 | 1.794 | 98.18 | 1.25 | 242 | 2.70 | 0.015 | — | 99.99 | 51.62 | 100.0 | 10.11 |
| 10 | 0.50 | 0.418 | 20.21 | 79.37 | 3.00 | 241 | 4.53 | 0.375 | 0.028 | 99.60 | 37.35 | 99.90 | 11.55 |
| 11 | 0.50 | 0.037 | 1.768 | 98.11 | 0.75 | 238 | 6.03 | 0.031 | — | 99.97 | 21.31 | 100.0 | 2.93 |
| 12 | 0.50 | 0.061 | 2.13 | 97.81 | 4.50 | 160 | 4.46 | 0.047 | 0.27 | 99.68 | 16.67 | 86.28 | — |
| 13 | 0.50 | 0.033 | 2.40 | 97.60 | 2.50 | 180 | 4.60 | 0.050 | 0.20 | 99.93 | — | 99.16 | — |
| 14 | 0.50 | 0.069 | 1.93 | 97.99 | 1.75 | 212 | 4.40 | 0.058 | — | 99.94 | 20.08 | 100.00 | 3.03 |
| 15 | 0.50 | 0.418 | 20.21 | 79.37 | 3.00 | 241 | 4.53 | 0.375 | 0.028 | 99.60 | 37.35 | 99.90 | 11.55 |
| 16 | 0.05 | 0.030 | 2.06 | 97.91 | 7.00 | 180 | 4.33 | 0.04 | 0.195 | 99.76 | — | 90.76 | 0.74 |
| 17 | 0.05 | 0.093 | 4.904 | 95.00 | 7.00 | 200 | 2.70 | 0.086 | 0.027 | 99.89 | 10.51 | 99.47 | — |
| 18 | 0.05 | 0.418 | 20.21 | 79.37 | 4.00 | 241 | 4.53 | 0.261 | 0.010 | 99.73 | 57.87 | 99.96 | 14.39 |

.*To total tetrachloropyridine isomers.

The data in Table II can be viewed in sections: Experiments 6—8 show the effect of catalyst concentration; experiments 9—11 show the effect of pressure on the reaction; experiments 12—18 show the effect of temperature on the reaction.

The negative numbers under "percent conversion" in Table II means that that particular isomer was being produced during the course of the reaction while the positive numbers, on the other hand, indicate that that particular isomer was being consumed during the course of the reaction and leading to reduced amounts of that material in the final product. In the above Runs, the small amount of 3,5-dichloropyridine present (not listed in the feed makeup) was converted to 2,3,5-trichloropyridine which in turn was converted to 2,3,4,5- and 2,3,5,6-tetrachloropyridine and pentachloropyridine. The 2,3,6-trichloropyridine (also not listes as part of the feed makeup) was converted to 2,3,5,6-tetrachloropyridine and pentachloropyridine. Additional reaction time would increase pentachloropyridine production and give substantially complete chlorination of 2,3,5-trichloropyridine, 2,3,4,5- and 2,3,4,6-tetrachloropyridine.

The dashes in Table II means that none of that particular isomer was present in the final reaction product.

These reaction mixtures were likewise resolved into pentachloropyridine and symmetrical tetrachloropyridine using distillative techniques.

Examples 19—30

In this series of experiments, a mixture of tetra- and pertachloropyridines was charged to a high pressure stainless steel autoclave, the reaction mixture melted, and one catalyst indicated in Table III added. The autoclave was subsequently sealed, heated to reaction temperature, and pressurized with a pod of chlorine entering through a top valve. Once pressurized, chlorine gas was then continuously sperged through the reaction mixture with stirring and analytical samples taken during the course of the reaction. The data from these experiments are summarized in Table III.

7

# TABLE III

## Effect of Various Catalysts

| Ex. | Catalyst | Wt. % Catalyst* | Feed – Percent Tetrachloro-pyridine Isomers 2,3,4,5 | 2,3,4,6 | 2,3,5,6 | Run Conditions Time hrs. | Temp °C | Pressure atm | Distribution In Product, Percent 2,3,4,5 | 2,3,4,6 | 2,3,5,6 | Percent Conversion 2,3,4,5 | 2,3,4,6 | 2,3,5,6 |
|---|---|---|---|---|---|---|---|---|---|---|---|---|---|---|
| 19 | $AlCl_3$ | 0.43 | 0.070 | 2.01 | 97.92 | 1.5 | 220 | 4.4 | 0.062 | — | 99.94 | 17.11 | 100.0 | 4.35 |
| 20 | $Al_2O_3$ | 1.43 | 0.030 | 1.745 | 98.22 | 2.5 | 240 | 4.46 | 0.019 | 0.063 | 99.91 | 37.63 | 98.34 | 0.16 |
| 21 | $Fe_2O_3$ | 5.02 | 0.034 | 1.814 | 9815 | 3.0 | 243 | 4.67 | 0.017 | — | 99.98 | 72.38 | 100.0 | 42.72 |
| 22 | Fe** | 0.49 | 0.180 | 12.69 | 87.13 | 3.0 | 243 | 4.46 | 0.140 | — | 99.85 | 53.71 | 97.67 | 13.52 |
| 23 | $SbCl_5$ | 0.90 | 0.069 | 1.930 | 97.99 | 1.25 | 222 | 4.4 | .0.060 | 0.899 | 99.04 | 16.01 | 55.01 | 2.38 |
| 24 | None | — | 0.136 | 2.676 | 97.19 | 4.0 | 203 | 4.4 | 0.107 | 2.896 | 96.99 | 22.22 | –6.99 | 1.34 |
| 25 | AlOCl | 0.987 | 0.088 | 8.948 | 90.96 | 2.0 | 220 | 2.7 | 0.177 | 0.055 | 99.75 | –75.4 | 99.5 | .4.33 |
| 26 | $AlCl_3$ on 13X molecular sieve | 0.421 | 0.141 | 9.634 | 90.23 | 1.0 | 220 | 2.7 | 0.148 | 0.118 | .99.73 | 10.42 | 98.95 | 5.65 |
| 27 | 9.9% $AlCl_3$ on silica gel | 0.610 | 0.098 | 9.421 | 90.48 | 2.0 | 220 | 2.7 | 0.154 | 0.011 | 99.84 | –34.7 | 99.9 | 5.40 |
| 28 | $AlCl_3$ on alumina | 0.109 | 0.129 | 8.695 | 91.16 | 2.0 | 220 | 2.7 | 0.204 | 0.059 | 99.74 | –42.1 | 99.4 | 1.74 |
| 29 | 22.9% $AlCl_3$ on graphite | 1.130 | 0.067 | 6.658 | 93.28 | 4.0 | 220 | 2.7 | 0.155 | 0.118 | 99.73 | –122.1 | 98.30 | –2.64 |
| 30 | $H_3PO_4$ (solid) | 8.07 | 0.115 | 8.330 | 91.55 | 8.0 | 220 | 2.7 | 0.148 | 0.218 | 99.63 | –15.3 | 97.7 | 2.47 |

*To total tetrachloropyridine isomers
**Steel wool

These reaction mixtures were likewise resolved into pentachloropridine and symmetrical tetrachloropyridine using distillative techniques.

The above examples show that the present process is very effective in resolving a complex mixture of chlorinated pyridines into a mixture consisting essentially of pentachloropyridine and symmetrical tetrachloropyridine which are easily resolved by conventional techniques. Various catalysts have been used in these examples and various other process parameters have been illustrated. Other catalysts and/or process parameters can be varied according to the above teaching.

The data in Table III shows the effect of the various catalysts in addition to ferric chloride; and experiment 24 shows the effect of no catalyst at all.

The negative numbers under "percent conversion" in Table III means that particular isomer was being produced during the course of the reaction. The positive numbers, on the other hand, indicate that that particular isomer was being consumed during the course of the reaction and leading to reduced amounts of that material in the final product.

The dashes in Table III means that none of that particular isomer was present in either the feed or the final reaction product.

### Claims

1. A process for recovering substantially pure 2,3,5,6-tetrachloropyridine from a mixture of chlorinated pyridines consisting of 2,3,5,6-tetrachloropyridine, at least one of 2,3,4,5-tetrachloropyridine and 2,3,4,6-tetrachloropyridine and, optionally, pentachloropyridine, and no more than minor amounts, if any, of monochloropyridine, 3,5-dichloropyridine, 2,3,5-trichloropyridine and 2,3,6-trichloropyridine and no other chloropyridine compounds, which process comprises

(a) reacting the mixture of chlorinated pyridines in liquid phase optionally in an inert solvent at a temperature above the melting point of the mixture under atmospheric or superatmospheric pressure with chlorine in the presence of up to 10 weight percent based on the weight of the chloropyridine reactant mixture of a catalyst which is o-phosphoric acid, boron trifluoride, aluminum chloride, aluminum oxychloride, chromium trichloride, ferric chloride, ruthenium chloride, aluminum bromide, aluminum oxybromide, chromium tribromide, ferric bromide, ruthenium bromide, aluminum oxide, ferric oxide, ferric acetylacetonate, antimony pentachloride optionally supported on a carrier, to obtain a mixture consisting essentially of 2,3,5,6-tetrachloropyridine and pentachloropyridine and

(b) recovering the 2,3,5,6-tetrachloropyridine by conventional techniques.

2. A process as claimed in Claim 1 wherein the catalyst is ferric chloride or aluminum chloride.

3. A process as claimed in Claim 1 or Claim 2 wherein the catalyst is present in an amount of from 0.05 to 0.5 weight percent, based on the weight of the first mixture of chlorinated pyridines.

4. A process as claimed in any one of the preceding claims wherein the reaction is effected at a temperature of from 150° to 300°C.

5. A process as claimed in Claim 4 wherein the reaction is effected at a temperature of from 200° to 250°C.

6. A process as claimed in any one of the preceding claims wherein the reaction is carried out at superatmospheric pressure in the absence of a solvent.

7. A process as claimed in any one of the preceding claims wherein the 2,3,5,6-tetrachloropyridine is recovered in step (b) by fractional distillation.

### Revendications

1. Procédé pour la récupération de la 2,3,5,6-tétrachloropyridine pratiquement pure à partir d'un mélange de pyridines chlorées constitué par la 2,3,5,6-tétrachloropyridine, au moins une des 2,3,4,5-tétrachloropyridine et 2,3,4,6-tétrachloropyridine et, éventuellement, par la pentachloropyridine, et seulement des quantités mineures, s'il y en a, de monochloropyridine, 3,5-dichloropyridine, 2,3,5-trichloropyridine et 2,3,6-trichloropyridine et pas d'autres composés chloropyridiques, procédé qui consiste

(a) à faire réagir le mélange de pyridines chlorées en phase liquide, éventuellement dans un solvant inerte, à une température supérieure au point de fusion du mélange, sous la pression atmosphérique ou sous une pression supérieure à celle-ci, avec du chlore en présence jusqu'à 10% en poids, par rapport au poids du mélange de réactifs chloropyridiques d'un catalyseur qui est l'acide orthophosphorique, le trifluorure de bore, le chlorure d'aluminium, l'oxychlorure d'aluminium, le trichlorure de chrome, le chlorure ferrique, le chlorure de ruthénium, le bromure d'aluminium, l'oxybromure d'aluminium, le tribromure de chrome, le bromure ferrique, le bromure de ruthénium, l'oxyde d'aluminium, l'oxyde ferrique, l'acétylacétonate ferrique, le pentachlorure d'antimoine, éventuellement supporté sur un support, pour obtenir un mélange constitué essentiellement par la 2,3,5,6-tétrachloropyridine et la pentachloropyridine et

(b) à récupérer la 2,3,5,6-tétrachloropyridine par les techniques classiques.

2. Procédé selon la revendication 1, dans lequel le catalyseur est le chlorure ferrique ou le chlorure d'aluminium.

3. Procédé selon la revendication 1 ou la revendication 2, dans lequel le catalyseur est présent à raison de 0,05 à 0,5% en poids par rapport au poids du premier mélange de pyridines chlorées.

4. Procédé selon l'une quelconque des revendications précédentes, dans lequel la réaction est effectuée à une température de 150° à 300°C.

5. Procédé selon la revendication 4, dans lequel la réaction est effectuée à une témperature de 200° à 250°C.

6. Procédé selon l'une quelconque des revendications précédentes, dans lequel la réaction est effectuée sous une pression supérieure à la pression atmosphérique en l'absence d'un solvant.

7. Procédé selon l'une quelconque des revendications précédentes, dans lequel la 2,3,5,6-tétrachloropyridine est récupérée dans le stade (b) par distillation fractionnée.

**Patentansprüche**

1. Verfahren zur Gewinnung von im wesentlichen reinent 2,3,5,6-Tetrachlorpyridin aus einer Mischung von chlorierten Pyridinen, die aus 2,3,5,6-Tetrachlorpyridin, mindestens einer der Verbindungen 2,3,4,5-Tetrachlorpyridin und 2,3,4,6-Tetrachlorpyridin und, ggf., Pentachlorpyridin und, wenn überhaupt, aus nicht mehr als kleinen Mengen Monochlorpyridin, 3,5-Dichlorpyridin, 2,3,5-Trichlorpyridin und 2,3,6-Trichlorpyridin und keinen anderen Chlorpyridinverbindungen besteht, dadurch gekennzeichnet, daß man

a) die Mischung der chlorierten Pyridine in flüssiger Phase, ggf. in einem inerten Lösungsmittel bei einer Temperatur oberhalb des Schmelzpunktes der Mischung unter atmosphärischem oder erhöhtem Druck mit Chlor in Gegenwart von bis zu 10 Gew.-%, bezogen auf das Gewicht der Chlorpyridin-Reaktionsmischung, eines Katalysators reagieren läßt, der o-Phosphorsäure, Bortrifluorid, Aluminiumchlorid, Aluminium-oxychlorid, Chromtrichlorid, Eisen (3)-Chlorid, Rutheniumchlorid, Aluminiumbromid, Aluminiumoxybromid, Chromtribromid, Eisen(3)-bromid, Rutheniumbromid, Aluminiumoxid, Eisen(3)-Oxid, Eisen(3)-Acetylacetonat, Antimonpentachlorid ist, und der sich ggf. auf einem Träger befindet, reagieren läßt, um eine Mischung zu erhalten, die im wesentlichen aus 2,3,5,6-Tetrachlorpyridin und Pentachlorpyridin besteht, und

b) das 2,3,5,6-Tetrachlorpyridin mittels konventioneller Verfahren gewinnt.

2. Verfahren nach Anspruch 1, dadurch gekennzeichnet, daß der Katalysator Eisen(3)-chlorid oder Aluminiumchlorid ist.

3. Verfahren nach Anspruch 1 oder 2, dadurch gekennzeichnet, daß der Katalysator in einer Menge von 0,05—0,5 Gew.-%, bezogen auf das Gewicht der ersten Mischung der chlorierten Pyridine, vorhanden ist.

4. Verfahren nach einem der verhergehenden Ansprüche, dadurch gekennzeichnet, daß die Reaktion bei einer Temperatur von 150—300°C durchgeführt wird.

5. Verfahren nach Anspruch 4, dadurch gekennzeichnet, daß die Reaktion bei einer Temperatur von 200—250°C durchgeführt wird.

6. Verfahren nach einem der vorhergehenden Ansprüche, dadurch gekennzeichnet, daß die Reaktion bei erhöhtem Druck in Abwesenheit eines Lösungsmittels durchgeführt wird.

7. Verfahren nach einem der vorhergehenden Ansprüche, dadurch gekennzeichnet, daß das 2,3,5,6-Tetrachlorpyridin in Stufe (b) durch fraktionierte Destillation gewonnen wird.